# EUROPEAN PATENT SPECIFICATION

(11) **EP 2 799 228 B1**
(45) Date of publication and mention of the grant of the patent: **14.10.2015**
(21) Application number: 13166524.2
(22) Date of filing: 03.05.2013
(51) Int. Cl.: B32B 27/12, B32B 38/14, B32B 38/00, A61F 13/15, B32B 37/20

(54) **Method of making a printed elastic laminate**
Verfahren zur Herstellung eines gedruckten elastischen Laminats
Procédé de fabrication d'un stratifié élastique imprimé

(43) Date of publication of application: 05.11.2014
(73) Proprietor: Mondi Gronau GmbH, 48599 Gronau (DE)
(72) Inventor: Homölle, Dieter, 48607 Ochtrup (DE); Schönbeck, Marcus, Dipl.-Ing., 33775 Versmold (DE)
(74) Representative: Albrecht, Rainer Harald

(56) References cited:
- DE-A1-102004 035 649
- US-A1- 2006 254 698
- US-A1- 2007 167 929

## Description

The invention relates to a method of making a printed, elastic laminate.

The invention is based on a method that envisions cutting an elastic film into strips that are laminated next to each other between two textile surface layers to form a laminate that is stretched transversely in areas rendered elastic by the strips relative to the web. The transverse stretching, which is also referred to as mechanical activation, improves the elastic properties of the laminate transversely of the direction of the material web (CD direction).

The textile surface layers can be made of a nonwoven, woven, or a knitted material. The strips can be configured in a single layer or a plurality of layers containing a sufficient quantity of an elastomeric polymer. The strips are glued or welded to the surface layers. One can die-cut elastic elements for hygiene products from the laminate, particularly elastic closure strips for diapers having an elastic middle region and adjacent thereto less elastic ends. The ends that are not elastic or less elastic are used for fastening hook-and-loop closure elements such as, for example, hook-fastener tapes thereto, and for attaching the elastic element to the inelastic regions of a diaper. The laminate is manufactured as a broad web that includes a plurality of laminated elastic strips. It is then possible to die-cut from the resulting multipurpose material the closure strips that are needed to make diapers.

A method of the kind as described above is known, for example, from EP-A1 686 209 or US-A-2006/254698.

WO 2011/045685 discloses a method of making a printed diaper manufactured from elastic closure elements and inelastic diaper sections. The elastic closure elements as well as the inelastic diaper sections are made of laminates that include a textile surface layer and a film liner or film carrier. The elastic closure elements as well as the inelastic part of the diaper carry an imprint whose printed images are harmonized at the edges of elastic and inelastic regions such that there results a uniform and attractive overall image.

WO 2008/070131 also discloses diapers having printed elastic and inelastic sections where the printed images are able to complement each other to form a uniform motif.

However, the job of printing the image onto the laminate using the method described above has not been satisfactorily resolved to date. If a previously printed textile surface layer is used for making the laminate, there exists the problem that the printed image previously applied to the textile surface is destroyed at least in part by tears and shifting of the fibers during the mechanical activation of the laminate and any later use by the consumer. Moreover, problems can result that are related to the positioning of the printed image that is a surface layer and the elastic strips that are added thereto. To be considered therein is the fact that different web tensions are transferred to the strips during cutting of the film into strips, advance of the web of elastic strips, and also during laminating; this can result in a change of strip width. If the printed image is made of a random printed motif having elements that are distributed across the entire print width, alignment errors of the elastic strips and the printed image are often not noticeable. However, if the regions made elastic by the strips are printed with, for example, a striped motif, optical inaccuracies are clear between the position of the strip and the printed motif if elastic elements manufactured from the laminate are extensively stretched during use, for example, when closing a diaper.

In view of this background, it is the object of the present invention to improve the appearance of the printed image on an elastic laminate that is made according to the method as described above.

The object of the invention and the solution for attaining the object of improving manufacture of an elastic laminate as described above met by a method according to claim 1.

According to the invention, the elastic film is printed with a motif made visible through the textile surface layer of the laminate before cutting the strips. Due to the fact that the elastic film is provided with the imprint, even while using the laminate that has been printed, correct alignment of the printed motif relative to the elastic region of the laminate is always ensured. The advantage therein lies in the fact that, when stretching the elastic strip, the printed image is evenly and reversibly stretched along with it. Furthermore, the printed motif is visible from the front side as well as from the back side of the laminate, for example, through a nonwoven textile surface layer, such that the laminate is optically equally attractive from the front as well as from the back. For example, the elastic film can be printed with a striped motif consisting of parallel, colored stripes extending in the direction of the web of the elastic film.

Known continuous printing methods can be used for printing the elastic film. Rotary printing processes are preferred that allow for printing the elastic film at high web speeds. The goal is web speeds of approximately 400 m/min. Gravure printing and flexography methods are advantageous processes, flexography being particularly preferred because it is possible to use one central cylinder for a plurality of color systems. Digital printing that transfers the printed image directly from a computer into a printing machine without the use of a static medium are not excluded. In particular, ink-jet printing methods are conceivable that generate a printed image by deflecting small ink drops.

According to a preferred embodiment of the invention, the elastic film is stretched transversely of the direction of the web before the printing process, then printed after elastic retraction, and subsequently cut into strips. The stretching of the elastic film constitutes a mechanical preactivation of a layer of the laminate and results in improved stretching behavior of the laminate. The preactivation of the elastic film has a positive effect on the course of the expansion force and provides easy stretching action of the laminate over a large area; and at an expansion limit that is determined by the preactivation of the elastic film and after which the expansion resistance increases strongly. The return behavior of the laminate after tension is removed can also be improved if the elastic film is preactivated by transverse stretching before laminating it into the laminate. Any preactivation of the elastic film cannot replace but can only supplement the mechanical activation of the laminate. Even when the elastic film is preactivated, it is still necessary for the laminate to be stretched transversely of the direction of the web in regions that are to be rendered elastic by laminated strips.

A preferred embodiment of the method according to the invention provides that the elastic film is stretched transversely of the web by more than 50% and has a width after reverse expansion that is greater than the starting width of the elastic film by 10% to 30% before it was stretched. The term "stretching" is used despite the fact that the expansion is not completely reversible but that some plastic deformation results in the film having a larger width following the reverse expansion. Later activation of the laminate essentially affects the structure of the textile surface layers. The transverse stretching of the preactivated elastic film, on the other hand, is for the most part reversible. The printed image that is applied to the preactivated elastic film thus does not undergo any further disadvantageous changes during subsequent activation of the laminate. Correspondingly, it is possible to improve the quality of the printed image on the elastic laminate if the elastic film is only printed following preactivation, during which the elastic film is expanded transversely and then released.

It is possible to use a stretch-rolling apparatus of profile rollers that mesh with each other for the stretching action of the elastic film and/or the laminate.

Preferably a polyolefin elastomer film is used as the elastic film. When using an polyolefin-elastomer-based elastic film, preactivation of the elastic film is especially advantageous.

In addition, it is also possible to use as an elastic film a single-layer or multilayer film having an elastomeric core layer made a material of styrene-isoprene-styrene block copolymers (SIS), styrene-butadiene-styrene block copolymers (SBS), styrene-ethylene-butylene-styrene block copolymers (SEBS), polyurethanes, ethylene copolymers, or polyether block amides.

After preactivation and printing, the elastic film is cut into strips. The strips are guided across a deflector and can be supplied as parallel strips to a laminator where the strips are laminated between the textile surface layers. The elastic strips are position at a transverse spacing from each other. The transverse spacing between the strips can be adjusted by the position of the deflector. In the gaps between the elastic strips, the surface layers are directly bonded to each other. It is within the scope of the invention to use reinforcement strips that are laminated between the elastic strips so as to reinforce the gaps between the elastic strips. It is thus possible to constitute elastic and inelastic regions inside the laminate.

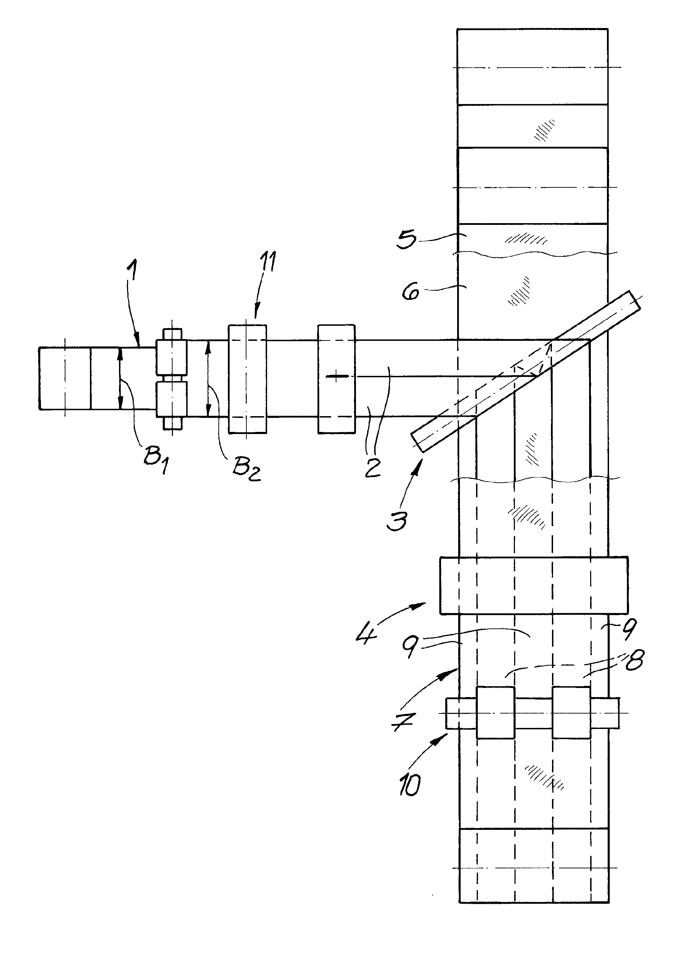

Below the invention will be illustrated in further detail with reference to an illustrated embodiment. The single figure is a schematic representation of a method of making a printed, elastic laminate.

With the method shown in the figure an elastic film 1 is cut into strips 2 that are guided across a deflector 3 and supplied to a laminator 4 as parallel strips. The strips 2 are laminated in a laminating direction 4 between textile surface layers 5 and 6 that are fed from above and below to the strips 2. The strips 2 and the textile surface layers 5 and 6 are glued together or connected to each other thermally. The view in the figure demonstrates that the elastic strips 2 are laminated at a spacing from each other between the surface layers 5 and 6 and that the textile surface layers 5 and 6 are directly connected to each other in gaps between the elastic strips 2. This way, elastic regions 8 and 10 as well as inelastic regions 9 are created in the laminate 7. The laminate is then supplied to an activator 10 in which the laminate 7 is stretched transversely at the regions 8 rendered elastic by the laminated strips 2 relative to the direction of the web. A stretch-roller apparatus having profile rollers that mesh with each other is used for stretching the laminate 7. Stretching modifies the textile structures of the surface layers, and the expansion property of the laminate 7 is improved in the CD direction, that is transversely of the longitudinal web direction. Following activation, the laminate is easily expandable in the CD direction by minimal force to an expansion limit that is determined by the activation.

The textile surface layers 5 and 6 are made of, in particular, nonwoven fabric; woven or knitted fabrics are also possible. A single-layer or multilayer elastomer film can be used as elastic film 1 having an elastomer core layer made of styrene-isoprene-styrene block copolymers, styrene-butadiene-styrene block copolymers, styrene-ethylene-butylene-styrene block copolymers, polyurethanes, ethylene copolymers, or polyether block amides. An elastic blown film made of a polyolefin elastomer is preferred.

Before cutting the film into strips 2, the elastic film 1 is printed in a printing station 11 with a motif that is visible through the textile surface layer 5 and 6 of the laminate 7. Printing is preferably done by a rotary printing method, particularly flexography. The printed motif can be, for example, a striped motif made of parallel colored stripes that extend in the longitudinal direction of the elastic film of the web.

Before printing, the elastic film 1 is stretched transversely of the web by more than 50%. Preferably, an expansion by 100% to 500% is effected relative to a starting width of the elastic film. After the elastic retraction, the elastic film 1 has a width B₂ that is larger by 10% to 30% than a starting width B₁ of the elastic film. Following the retraction, the elastic film 1 is printed and subsequently cut into strips 2. Expanding and/or stretching the elastic film 1 constitutes preactivation. Preactivation of the elastic film has considerable advantages with regard to the expansion values of the laminate 7. Due to preactivation of the elastic film 1 prior to the printing process, it is also possible to improve the printed image of the elastic laminate 7, the reason for this being that during stretching of the laminate 7, the printed image is evenly and reversibly expanded along with the laminate, and preactivation of the elastic film 1 results in the laminate 7 completely resetting itself following stretching in the activation apparatus 10.

## Claims

1. A method of making a printed, elastic laminate, wherein an elastic film (1) is cut into strips (2),
the strips (2) are laminated next to each other between two surface layers (5, 6), and
the thus constituted laminate (7) is stretched in regions (8) rendered elastic transversely of the web by the strips (2) laminated therein, **characterized in that** the elastic film (1) is printed with a motif that is visible through the textile surface layer (5, 6) of the laminate (7) before being cut into strips (2).

2. The method according to claim 1, **characterized in that** the elastic film (1) is printed with a striped motif made of parallel colored strips extending in the direction of the web of the elastic film (1).

3. The method according to claim 1 or 2, **characterized in that** the elastic film (1) is printed by flexography.

4. The method according to claim 1 or 2, **characterized in that** the elastic film (1) is printed by gravure printing or a digital printing.

5. The method according to any one of the claims 1 to 4, **characterized in that**, prior to printing, the elastic film (1) is stretched transversely of the direction of the web and printed after an elastic retraction.

6. The method according to claim 5, **characterized in that** the elastic film (1) is expanded transversely of the direction of the web by more than 50% to have a width (B₂) after elastic retraction that is larger by 10% to 30% than a starting width (B₁) of the elastic film (1) prior to stretching.

7. The method according to any one of the claims 1 to 6, **characterized in that** a stretch-roller apparatus made of profile rollers that mesh with each other is used for stretching the elastic film (1) and/or laminate (7).

8. The method according to any one of the claims 1 to 7, **characterized in that** a film made of a polyolefin elastomer is used as the elastic film (1).

9. The method according to any one of the claims 1 to 8, **characterized in that** a single-layer or multilayer film is used as the elastic film (1) and has an elastomer core layer made from styrene-isoprene-styrene block copolymers, styrene-ethylene-butylene-styrene-block copolymers, polyurethanes, ethylene-copolymers or polyether block amides.

10. The method according to any one of the claims 1 to 9, **characterized in that** the strips (2) are guided across a deflector (3) and supplied as parallel strips (4)to a laminator inside which the strips (2) are laminated between the textile surface layers (5, 6).

11. The method according to any one of the claims 1 to 10, **characterized in that** the elastic strips (2) are laminated at a spacing from each other between the surface layers (5, 6) to form elastic and inelastic regions (8, 9) in the laminate (7).

## Patentansprüche

1. Verfahren zur Herstellung eines bedruckten, elastischen Verbundstoffes, wobei
eine elastische Folie (1) in Folienstreifen (2) geschnitten wird,
die Folienstreifen (2) nebeneinander zwischen zwei textile Deckschichten (5, 6) einkaschiert werden und
der so gebildete Verbundstoff (7) in Bereichen (8), die durch die einkaschierten Folienstreifen (2) elastisch sind, quer zur Bahnrichtung gereckt wird,
**dadurch gekennzeichnet, dass** die elastische Folie (1) vor dem Auftrennen in die Folienstreifen (2) mit einem Motiv bedruckt wird, welches durch die textile Deckschicht (5, 6) des Verbundstoffes (7) durchscheint.

2. Verfahren nach Anspruch 1, **dadurch gekennzeichnet, dass** die elastische Folie (1) mit einem Streifenmotiv aus parallelen farbigen Streifen, die sich in Bahnrichtung der elastischen Folie (1) erstrecken, bedruckt wird.

3. Verfahren nach Anspruch 1 oder 2, **dadurch gekennzeichnet, dass** die elastische Folie (1) mittels eines Flexodruckverfahrens bedruckt wird.

4. Verfahren nach Anspruch 1 oder 2, **dadurch gekennzeichnet, dass** die elastische Folie (1) mittels eines Tiefdruckverfahrens oder mittels eines Digitaldruckverfahrens bedruckt wird.

5. Verfahren nach einem der Ansprüche 1 bis 4, **dadurch gekennzeichnet, dass** die elastische Folie (1) vor dem Bedrucken quer zur Bahnrichtung gedehnt und nach einer elastischen Rückdehnung bedruckt wird.

6. Verfahren nach Anspruch 5, **dadurch gekennzeichnet, dass** die elastische Folie (1) quer zur Bahnrichtung um mehr als 50 % gedehnt wird und nach der elastischen Rückdehnung eine Breite (B₂) aufweist, die um 10% bis 30 % größer ist als die Anfangsbreite (B₁) der elastischen Folie (1) vor ihrer Verstreckung.

7. Verfahren nach einem der Ansprüche 1 bis 6, **dadurch gekennzeichnet, dass** zum Verstrecken der elastischen Folie (1) und/oder des Verbundstoffes (7) eine Reckwalzenanordnung aus ineinandergreifenden Profilwalzen verwendet wird.

8. Verfahren nach einem der Ansprüche 1 bis 7, **dadurch gekennzeichnet, dass** als elastische Folie (1) eine Folie aus einem Polyolefinelastomer verwendet wird.

9. Verfahren nach einem der Ansprüche 1 bis 8, **dadurch gekennzeichnet, dass** als elastische Folie (1) eine einschichtige Folie oder eine mehrschichtige Folie mit einer elastomeren Kernschicht aus der Gruppe der Styrol-Isopren-Styrol-Blockcopolymere, Styrol-Butadien-Styrol-Blockcopolymere, Styrol-Ethylen/ Butylen-Styrol-Blockcopolymere, Polyurethane, Ethylen-Copolymere oder Polyether-Blockamide verwendet wird.

10. Verfahren nach einem der Ansprüche 1 bis 9, **dadurch gekennzeichnet, dass** die Folienstreifen (2) über Umlenkeinrichtungen (3) geführt und als parallele Streifen einer Kaschiereinrichtung (4) zugeführt werden, in der die Folienstreifen (2) zwischen die textilen Deckschichten (5, 6) einkaschiert werden.

11. Verfahren nach einem der Ansprüche 1 bis 10, **dadurch gekennzeichnet, dass** die elastischen Folienstreifen (2) im Abstand zueinander zwischen die Deckschichten (5, 6) einkaschiert werden und im Verbundstoff (7) elastische und nicht elastische Bereiche (8, 9) gebildet werden.

## Revendications

1. Procédé de fabrication d'un laminé élastique imprimé, dans lequel un film élastique (1) est découpé en bandes (2),
les bandes (2) sont laminées l'une à côté de l'autre entre deux couches surfaciques (5, 6) et
le laminé (7) ainsi constitué est étendu en zones (8) rendues élastiques transversalement à la feuille continue par les bandes (2) laminées dessus, **caractérisé en ce que** le film élastique (1) est imprimé avec un motif qui est visible à travers la couche surfacique textile (5, 6) du laminé (7) avant d'être découpé en bandes (2).

2. Procédé selon la revendication 1, **caractérisé en ce que** le film élastique (1) est imprimé avec un motif à rayures fait de bandes colorées parallèles s'étendant dans la direction de la feuille continue du film élastique (1).

3. Procédé selon la revendication 1 ou 2, **caractérisé en ce que** le film élastique (1) est imprimé par flexographie.

4. Procédé selon la revendication 1 ou 2, **caractérisé en ce que** le film élastique (1) est imprimé par gravure ou impression numérique.

5. Procédé selon l'une quelconque des revendications 1 à 4, **caractérisé en ce qu'**avant l'impression, le film élastique (1) est étiré transversalement à la direction de la feuille continue puis imprimé après retrait élastique.

6. Procédé selon la revendication 5, **caractérisé en ce que** le film élastique (1) est étiré transversalement à la direction de la feuille continue de plus de 50 % pour présenter une largeur (B₂) après retrait élastique qui est supérieure de 10 % à 30 % à une largeur de départ (B₁) du film élastique (1) avant l'étirement.

7. Procédé selon l'une quelconque des revendications 1 à 6, **caractérisé en ce qu'**un appareillage de rouleau d'étirement fait de rouleaux profilés qui s'entremêlent est utilisé pour étirer le film élastique (1) et/ou le laminé (7).

8. Procédé selon l'une quelconque des revendications 1 à 7, **caractérisé en ce qu'**un film fait d'élastomère polyoléfine est utilisé en tant que ledit film élastique (1).

9. Procédé selon l'une quelconque des revendications 1 à 8, **caractérisé en ce qu'**un film monocouche ou multi-couches est employé en tant que film élastique (1) et présente une couche à noyau élastomère faite de copolymères séquencés styrène-isoprène-styrène, de copolymères séquencés styrène-éthylène-butylène-styrène, de polyuréthanes, de copolymères-éthylènes ou de polyéther bloc amides.

10. Procédé selon l'une quelconque des revendications 1 à 9, **caractérisé en ce que** les bandes (2) sont guidées à travers un déflecteur (3) et alimentées en tant que bandes parallèles (4) vers une contrecolleuse à l'intérieur de laquelle les bandes (2) sont laminées entre les couches surfaciques textiles (5, 6).

11. Procédé selon l'une quelconque des revendications 1 à 10, **caractérisé en ce que** les bandes élastiques (2) sont laminées avec un espacement l'une de l'autre entre les couches surfaciques (5, 6) pour former des zones élastiques et inélastiques (8, 9) dans le laminé (7).
